# EUROPEAN PATENT SPECIFICATION

(11) **EP 0 984 013 B1**
(45) Date of publication and mention of the grant of the patent: **30.06.2004**
(21) Application number: 99203367.0
(22) Date of filing: 20.02.1992
(51) Int. Cl.: C07D 411/04, A61K 31/505, C12P 41/00, C12P 19/38, C12P 17/16, C07H 19/06, C07H 19/10

(54) **Antiviral activity and resolution of 2-hydroxymethyl-5-(5-fluorocytosin-1-yl)-1, 3-oxathiolane**
Antivirus Wirkung und Auflösung von 2-Hydroxymethyl-5-(5-fluorocytosin-1-yl)-1,3-oxathiolane
Activité antivirale et résolution de 2-hydroxymethyl-5-(5-fluorocytosin-1-yl)-1,3-oxathiolane

(30) Priority: 22.02.1991 US 659760; 26.07.1991 US 736089; 12.02.1992 US 831153
(43) Date of publication of application: 08.03.2000
(62) Divisional of application: 92908027.3
(73) Proprietor: EMORY UNIVERSITY, Atlanta, GA 30322 (US)
(72) Inventor: Liotta, Dennis C., Stone Mountain, GA 30088 (US); Schinazi, Raymond F., Decatur, GA 30333 (US); Choi, Woo-Baeg, Atlanta, GA 30339 (US)
(74) Representative: Hallybone, Huw George

(56) References cited:
- EP-A- 0 382 526
- WO-A-91/11186
- SHIN LIAN DOONG ET AL.: "INHIBITION OF THE REPLICATION OF HEPATITIS B VIRUS" PROCEEDINGS OF THE NATIONAL ACADEMY OF SCIENCES OF USA., vol. 88, no. 19, October 1991 (1991-10), pages 8495-9, XP002132311 NATIONAL ACADEMY OF SCIENCE. WASHINGTON., US ISSN: 0027-8424

## Description

### Background of the Invention

This invention is in the area of biologically active nucleosides, and specifically includes a method for the resolution and use of the (-)-β-L and (+)-β-D enantiomers of FTC.

In 1981, acquired immune deficiency syndrome (AIDS) was identified as a disease that severely compromises the human immune system, and that almost without exception leads to death. In 1983, the etiological cause of AIDS was determined to be the human immunodeficiency virus (HIV). By December of 1990, the World Health Organization estimated that between 8 and 10 million people worldwide were infected with HIV, and of that number, between 1,000,000 and 1,400,000 were in the U.S.

In 1985, it was reported that the synthetic nucleoside 3'-azido-3'-deoxythymidine (AZT) inhibits the replication of human immunodeficiency virus. Since then, a number of other synthetic nucleosides, including 2',3'-dideoxyinosine (DDI), 2',3'-dideoxycytidine (DDC), 3'-fluoro-3'-deoxythymidine (FLT), and 2',3'-dideoxy-2',3'-didehydrothymidine (D4T), have been proven to be effective against HIV. A number of other 2',3'-dideoxynucleosides have been demonstrated to inhibit the growth of a variety of viruses in vitro. It appears that, after cellular phosphorylation to the 5'-triphosphate by cellular kinases, these synthetic nucleosides are incorporated into a growing strand of viral DNA, causing chain termination due to the absence of the 3'-hydroxyl group.

The success of various 2',3'-dideoxynucleosides in inhibiting the replication of HIV in vivo or in vitro has led a number of researchers to design and test nucleosides that substitute a heteroatom for the carbon atom at the 3'-position of the nucleoside. Norbeck, et al., disclose that (±)-1-[(2β,4β)-2-(hydroxymethyl)-4-dioxolanyl]thymine (referred to as (±)-dioxolane-T) exhibits a modest activity against HIV (EC₅₀ of 20 µm in ATH8 cells), and is not toxic to uninfected control cells at a concentration of 200 µM. Tetrahedron Letters 30 (**46**), 6246, (1989). European Patent Application Publication No. 0 337 713 and U.S. Patent No. 5,041,449, assigned to IAF BioChem International, Inc., disclose 2-substituted-4-substituted-1,3-dioxolanes that exhibit antiviral activity.

U.S. Patent No. 5,047,407 and European Patent Application Publication No. 0 382 526, also assigned to IAF Biochem International, Inc. disclose a number of 2-substituted-5-substituted-1,3-oxathiolane nucleosides with antiviral activity, and specifically report that the racemic mixture (about the C4'-position) of the C1'-β isomer of 2-hydroxymethyl-5-(cytosin-1-yl)-1,3-oxathiolane (referred to below as (±)-BCH-189) has approximately the same activity against HIV as AZT, and no cellular toxicity at the tested levels. (±)-BCH-189 has also been found to inhibit the replication of AZT-resistant HIV isolates in vitro from patients who have been treated with AZT for longer than 36 weeks.

WO-A-92/15308 discloses the use of 1-(2-hydroxymethyl)-1,3-oxathiolan-5-yl)-5-fluorocytosine and pharmaceutically acceptable derivatives thereof for the treatment of hepatitis B virus infections.

Another virus that causes a serious human health problem is the hepatitis B virus (referred to below as "HBV"). HBV is second only to tobacco as a cause of human cancer. The mechanism by which HBV induces cancer is unknown, although it is postulated that it may directly trigger tumor development, or indirectly trigger tumor development through chronic inflammation, cirrhosis, and cell regeneration associated with the infection.

After a two to six month incubation period in which the host is unaware of the infection, HBV infection can lead to acute hepatitis and liver damage, that causes abdominal pain, jaundice, and elevated blood levels of certain enzymes. HBV can cause fulminant hepatitis, a rapidly progressive, often fatal form of the disease in which massive sections of the liver are destroyed.

Patients typically recover from acute hepatitis. In some patients, however, high levels of viral antigen persist in the blood for an extended, or indefinite, period, causing a chronic infection. Chronic infections can lead to chronic persistent hepatitis. Patients infected with chronic persistent HBV are most common in developing countries. By mid-1991, there were approximately 225 million chronic carriers of HBV in Asia alone, and worldwide, almost 300 million carriers. Chronic persistent hepatitis can cause fatigue, cirrhosis of the liver, and hepatocellular carcinoma, a primary liver cancer.

In western industrialized countries, high risk groups for HBV infection include those in contact with HBV carriers or their blood samples. The epidemiology of HBV is very similar to that of acquired immune deficiency syndrome, which accounts for why HBV infection is common among patients with AIDS or AIDS-related complex. However, HBV is more contagious than HIV.

A human serum-derived vaccine has been developed to immunize patients against HBV. While it has been found effective, production of the vaccine is troublesome because the supply of human serum from chronic carriers is limited, and the purification procedure is long and expensive. Further, each batch of vaccine prepared from different serum must be tested in chimpanzees to ensure safety. Vaccines have also been produced through genetic engineering. Daily treatments with a-interferon, a genetically engineered protein, has also shown promise. However, to date there is no known pharmaceutical agent that effectively inhibits the replication of the virus.

To market a nucleoside for pharmaceutical purposes, it must not only be efficacious with low toxicity, it must also be cost effective to manufacture. An extensive amount of research and development has been directed toward new, low cost processes for large scale nucleoside production. 2',3'-Dideoxynucleosides are currently prepared by either of two routes: derivatization of an intact nucleoside or condensation of a derivatized sugar moiety with a heterocyclic base. Although there are numerous disadvantages associated with obtaining new nucleoside analogues by modifying intact nucleosides, a major advantage of this approach is that the appropriate absolute stereochemistry has already been set by nature. However, this approach cannot be used in the production of nucleosides that contain either nonnaturally occurring bases or nonnaturally occurring carbohydrate moieties (and which therefore are not prepared from intact nucleosides), such as 1,3-oxathiolane nucleosides and 1,3-dioxolane nucleosides.

When condensing a carbohydrate or carbohydrate-like moiety with a heterocyclic base to form a synthetic nucleoside, a nucleoside is produced that has two chiral centers (at the C1' and C4'-positions), and thus exists as a diastereomeric pair. Each diastereomer exists as a set of enantiomers. Therefore, the product is a mixture of four enantiomers.

It is often found that nucleosides with nonnaturally-occurring stereochemistry in either the C1' or the C4'-positions are less active than the same nucleoside with the stereochemistry as set by nature. For example, Carter, et al., have reported that the concentration of the (-)-enantiomer of carbovir (2',3'-didehydro-2',3'-dideoxyguanosine) in cell culture required to reduce the reverse transcriptase activity by 50% (EC₅₀) is 0.8 µM, whereas the EC₅₀ for the (+)-enantiomer of carbovir is greater than 60 µM. Antimicrobial Agents and Chemotherapy, **34:6**, 1297-1300 (June 1990).

PCT International Publication No. WO 91/11186 discloses that 1,3-oxathiolane nucleosides can be prepared with high diastereoselectivity (high percentage of nucleoside with a B configuration of the bond from the C1'-carbon to the heterocyclic base) by careful selection of the Lewis acid used in the condensation process. It was discovered that condensation of a 1,3-oxathiolane nucleoside with a base occurs with almost complete β-stereospecificity when stannic chloride is used as the condensation catalyst. Other Lewis acids provide low (or no) C1'-β selectivity or simply fail to catalyze the reactions.

In light of the fact that acquired immune deficiency syndrome, AIDS-related complex, and hepatitis B virus have reached epidemic levels worldwide, and have tragic effects on the infected patient, there remains a strong need to provide new effective pharmaceutical agents to treat these diseases that have low toxicity to the host.

There is also a need to provide a cost effective, commercially viable method to produce pharmaceutically important nucleosides, and specifically attain β-stereospecificity in the C4'-position of synthetic nucleosides prepared by condensing a carbohydrate-like moiety with a base.

Therefore, it is an object of the present invention to provide a method and composition for the treatment of human patients infected with HIV.

It is another object of the present invention to provide a method and composition for the treatment of human patients or other host animals infected with HBV.

It is still another object of the present invention to provide enantiomerically enriched 1,3-oxathiolane nucleosides.

It is still another object of the present invention to provide a method for the resolution of C4'-enantiomers of 1,3-oxathiolane nucleosides.

### Summary of the Invention

According to one aspect of the present invention, there is provided a process for obtaining (-)-β-L-2-hydroxymethyl-5-(5-fluorocytosin-1-yl)-1,3-oxathiolane, comprising resolving a mixture of (-) and (+)-cis-2-hydroxymethyl-5-(5-fluoro-cytosin-1-yl)-1,3-oxathiolane by exposing the mixture to cytidine-deoxycytidine deaminase.

It has been discovered that 2-hydroxymethyl-5-(5-fluorocytosin-1-yl)-1,3-oxathiolane ("FTC"), exhibits surprisingly high activity against human immunodeficiency virus with very low host cell toxicity. It has also been discovered that FTC exhibits very significant activity against HBV, and therefore can be used to treat patients who have a variety of illnesses associated with HBV infection.

Toxicity and pharmacokinetic studies confirm the usefulness of FTC as an antiviral agent for pharmaceutical administration. FTC and its enantiomers are nontoxic to peripheral human bone marrow cells at concentrations up to 50 µM and other cell lines at concentrations up to 200 µM. FTC-TP is a major intracellular metabolite in PBMC and HepG2 cells. FTC-TP competitively inhibits HIV-1 reverse transcriptase (RT) with a K₁ of 0.2 µM using a poly(I)oligo(dC) template-primer. Using sequencing analysis, FTC-TP can be shown to be a potent DNA chain terminator when HIV-RT is used (C-stops).

Chronic treatment with FTC is not toxic to rodents, even at oral doses of 85 mg/kg per day for at least two months. The pharmacokinetics of FTC in rhesus monkeys indicates high oral bioavailability (approximately 73 ± 6%) and a plasma terminal half life of approximately 1.34 ± 0.18 (mean of oral and I.V. administration).

A process for the resolution of a racemic mixture of nucleoside enantiomers, including the racemic mixture of FTC, is also disclosed that includes the step of exposing the racemic mixture to an enzyme that preferentially catalyzes a reaction in one of the enantiomers. The process can be used to resolve a wide variety of nucleosides, including pyrimidine and purine nucleosides that are optionally substituted in the carbohydrate moiety or base moiety. The process can also be used to resolve nucleoside derivatives that contain additional heteroatoms in the carbohydrate moiety, for example, (±)-FTC and (±)-BCH-189. The resolution of nucleosides can be performed on large scale at moderate cost.

Using methods described herein, FTC was resolved into its (+)-β-D and (-)-β-L enantiomers. The (-)-β-L-enantiomer appears to be more potent that the (+)-β-D-enantiomer against HIV, HBV, and SIV. The (+)-enantiomer of FTC is also active against HIV, HBV, and SIV.

### Brief Description of the Figures

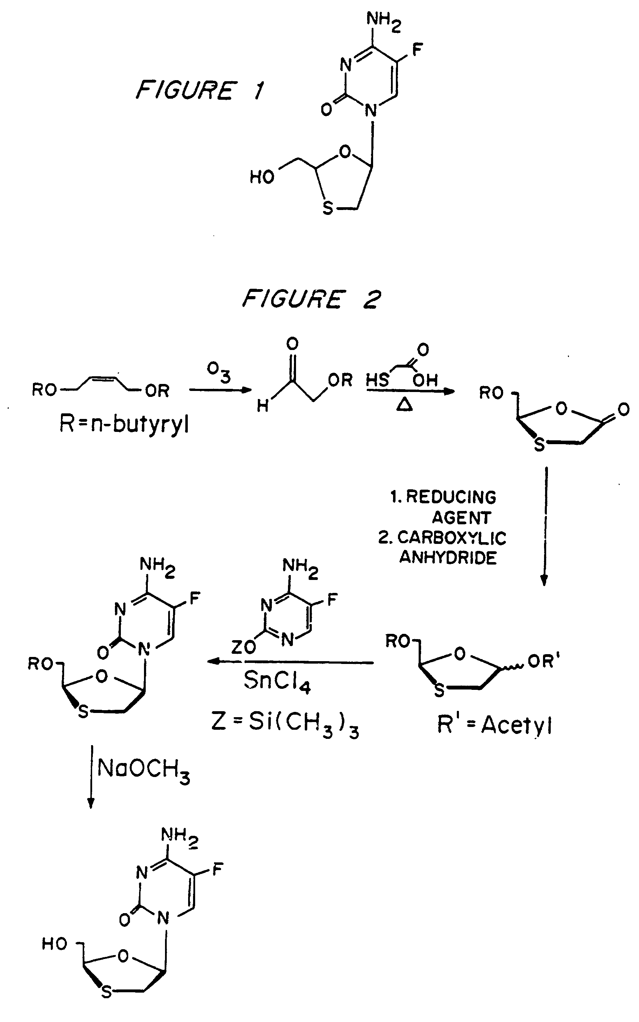

Figure 1 is an illustration of the chemical structure of 2-hydroxymethyl-5-(5-fluorocytosin-1-yl)-1,3-oxathiolane ("FTC").
Figure 2 is an illustration of a method for the preparation of 2-hydroxymethyl-5-(5-fluorocytosin-1-yl)-1,3-oxathiolane.
Figure 3 is a flow chart of the specificity of alkaline phosphatase and snake venom phosphodiesterase for the (+) and (-) enantiomers of FTC.
Figure 4 is a graph indicating the progress of lipase-catalyzed hydrolysis of the 5'-butyryl ester of FTC over time using the enzymes Amano PS-800® (-open square-) and PLE (-open circle with dot-).
Figure 5 is a graph of the effect of concentration (µM) of racemic and enantiomerically enriched FTC (prepared by the method of Example 3) versus the percent inhibition of human PBM cells infected with HIV-1. ((-darkened circle-, (±)-FTC), (-open circle-,(-)-PTC), (-darkened square-, (+)-FTC).
Figure 6 is a graph of the effect of concentration (µM) of racemic and enantiomerically enriched FTC (prepared by method of Example 2) on the percent inhibition of human PBM cells infected with HIV-1. ((-darkened circle-, (±)-FTC), (-open circle-,(-)-FTC), (-darkened square-,(+)-FTC).
Figure 7 is a graph of the uptake of tritiated (±)-FTC in human PBM cells (average of two determinations) in time (hours) versus pmol/10⁶ cells.
Figure 8 is a graph of the egress of radiolabeled (±)-FTC from human PBM cells, measured in hours versus pmol/10⁶ cells.
Figure 9 illustrates the presence of [³H]-(±)-FTC and its phosphorylated derivatives in human HepG-2 cells (average of two determinations) incubated in media containing 10 µM [³H]-(±)-FTC, measured in pmol/10⁶ cells over time.
Figure 10 illustrates the egress of [³H]-(±)-FTC and its phosphorylated derivatives in human HepG2 in pmol/10⁶ cells over time cells after pulsing cells with 10 µM [³H]-(±)-FTC (700 DPM/pmole) for 24 hours, and evaluating the concentration of compound 24 hours after removal.
Figure 11 illustrates the decrease in the combined concentration of [³H]-(±)-FTC and its phosphorylated derivatives from human HepG2 cells after incubation with 10 µM [³H]-(±)-FTC (700 DPM/pmole) for 24 hours, in pmol/10⁶ cells over time.
Figure 12 is a graph of the effect of the enantiomers of FTC on colony formation of granulocyte-macrophage precursor cells, as measured in percent survival versus concentration in µM ((-)-FTC, open circle; (+)-FTC, darkened circle; AZT, darkened square.

### Detailed Description of the Invention

As used herein, the term "enantiomerically enriched nucleoside" refers to a nucleoside composition that includes at least 95% of a single enantiomer of that nucleoside.

As used herein, the term FTC refers to 2-hydroxymethyl-5-(5-fluorocytosin-1-yl)-1,3-oxathiolane (the racemic form or enantiomers), also referred to as 2'-deoxy-5-fluoro-3'-thiacytidine.

As used herein, the term (±)-FTC refers to (±)-β-D,L-2-hydroxymethyl-5-(5-fluorocytosin-1-yl)-1,3-oxathiolane.

As used herein, the term (-)-FTC refers to (-)-B-L-2-hydroxymethyl-5-(5-fluorocytosin-1-yl)-1,3-oxathiolane

As used herein, the term (+)-FTC refers to (+)-β-D-2-hydroxymethyl-5-(5-fluorocytosin-1-yl)-1,3-oxathiolane.

As used herein, the terms FTC-MP, FTC-DP, and FTC-TP refer to the monophosphate, diphosphate, and triphosphate of FTC, respectively.

As used herein, the term BCH-189 refers to 2-hydroxymethyl-5-(cytosin-1-yl)-1,3-oxathiolane.

As used herein, the term "preferential enzyme catalysis" refers to catalysis by an enzyme that favors one substrate over another.

As used herein, a leaving group means a functional group that forms an incipient carbonation when it separates from the molecule that it is attached to.

The invention as disclosed herein may be used in a method and composition for the treatment of HIV and HBV infections, and other viruses replicating in like manner, in humans or other host animals. Such methods include administering an effective amount of the (-)-β-L enantiomer of 2-hydroxymethyl-5-(5-fluorocytosin-1-yl)-1,3-oxathiolane, in a pharmaceutically acceptable carrier. As shown below, this compound possesses antiretroviral activity, such as anti-HIV-1, anti-HIV-2 and anti-simian immunodeficiency virus (anti-SIV) activity.

(-)-β-L-2-hydroxymethyl-5-(5-fluorocytosin-1-yl)-1,3-oxathiolane and pharmaceutically acceptable formulations containing this compound are useful in the prevention and treatment of HIV infections and other related conditions such as AIDS-related complex (ARC), persistent generalized lymphadenopathy (PGL), AIDS-related neurological conditions, anti-HIV antibody positive and HIV-positive conditions, Kaposi's sarcoma, thrombocytopenia purpurea and opportunistic infections. In addition, these compounds or formulations can be used prophylactically to prevent or retard the progression of clinical illness in individuals who are anti-HIV antibody or HIV-antigen positive or who have been exposed to HIV.

(-)-β-L-2-hydroxymethyl-5-(5-fluorocytosin-1-yl)-1,3-oxathiolane and pharmaceutically acceptable formulations containing this compounds are also useful in the prevention and treatment of HBV infections and other related conditions such as anti-HBV antibody positive and HBV-positive conditions, chronic liver inflammation caused by HBV, cirrhosis, acute hepatitis, fulminant hepatitis, chronic persistant hepatitis, and fatigue. This compounds or formulations can also be used prophylactically to prevent or retard the progression of clinical illness in individuals who are anti-HBV antibody or HBV-antigen positive or who have been exposed to HBV.

In summary, the present invention includes the following features: a process for the preparation of a (-) or (+) enantiomer of 2-hydroxymethyl-5-(5-fluorocytosin-1-yl)-1,3-oxathiolane which comprises subjecting the compound or derivative (e.g. 5'-ester) thereof in the form of a mixture of (-) and (+) enantiomers to conditions or reacting with reagents serving to separate the enantiomers and if necessary converting the resulting derivative to the parent compound.

Resolution of the (±)-enantiomers can be accomplished as specified in detail in Section III. below.

### III. Resolution of Nucleoside Enantiomers

A method is provided herein for the resolution of racemic mixtures of nucleoside (+) and (-) enantiomers, of FTC. The method can also be used to resolve racemic mixtures of carbohydrates or carbohydrate-like moieties, such as derivatives of 1,3-oxathiolane and 1,3-dioxolane. The method involves the use of an enzyme that preferentially catalyzes a reaction of one enantiomer in a racemic mixture. The reacted enantiomer is separated from the unreacted enantiomer on the basis of the new difference in physical structure. Given the disclosure herein, one of skill in the art will be able to choose an enzyme that is selective for the nucleoside enantiomer of choice (or selective for the undesired enantiomer, as a method of eliminating it), by selecting one of the enzymes discussed below or by systematic evaluation of other known enzymes. Given this disclosure, one of skill in the art will also know how to modify the substrate as necessary to attain the desired resolution. Through the use of either chiral NMR shift reagents, polarimetry, or chiral HPLC, the optical enrichment of the recovered ester can be determined.

The following examples further illustrate the use of enzymes to resolve racemic mixtures of enantiomers. Other known methods of resolution of racemic mixtures can be used in combination with the method of resolution disclosed herein. All of these modifications are considered within the scope of the invention.

### Resolution of Nucleoside Enantiomers with Cytidine-Deoxycytidine Deaminase

In the present invention, cytidine-deoxycytidine deaminase is used to resolve racemic mixtures of 2-hydroxymethyl-5-(cytosin-1-yl)-1,3-oxathiolane and its derivatives, including 2-hydroxymethyl-5-(5-fluoro-cytosin-1-yl)-1,3-oxathiolane. The enzyme catalyses the deamination of the cytosine moiety to a uracil. It has been discovered that one of the enantiomers of 1,3-oxathiolane nucleosides is a preferred substrate for cytidine-deoxycytidine deaminase. The enantiomer that is not converted to a uracil derivative (and therefore is still basic) is extracted from the solution with an acidic solution. Care should be taken to avoid strong acidic solutions (pH below 3.0), that may cleave the oxathiolane ring.

Cytidine-deoxycytidine deaminase can be isolated from rat liver or human liver, or expressed from recombinant sequences in a procaryotic system such as in *E. coli*.

The method of resolution of cytidine nucleoside enantiomers using cytidine-deoxycytidine deaminase can be used as the sole method of resolution or can be used in combination with other methods of resolution, including resolution by enzymatic hydrolysis of 5'-o-nucleoside esters as described below.

### Resolution Based on Hydrolysis of C5'-Nucleoside Esters

In one embodiment, the method includes reacting the C5'-hydroxyl group of a mixture of nucleoside racemates with an acyl compound to form C5'-esters in which the nucleoside is in the "carbinol" end of the ester. The racemic mixture of nucleoside C5'-esters is then treated with an enzyme that preferentially cleaves, or hydrolyses, one of the enantiomers and not the other, in a given time period.

An advantage of this method is that it can be used to resolve a wide variety of nucleosides, including pyrimidine and purine nucleosides that are optionally substituted in the carbohydrate moiety or base moiety. The method can also be used to resolve nucleoside derivatives that contain additional heteroatoms in the carbohydrate moiety, for example, FTC and BCH-189. The broad applicability of this method resides in part on the fact that

although the carbinol portion of the ester plays a role in the ability of an enzyme to differentiate enantiomers, the major recognition site for these enzymes is in the carboxylic acid portion of the ester. Further, one may be able to successfully extrapolate the results of one enzyme/substrate study to another, seemingly-different system, provided that the carboxylic acid portions of the two substrates are the same or substantially similar.

Another advantage of this method is that it is regioselective. Enzymes that hydrolyse esters typically do not catalyze extraneous reactions in other portions of the molecule. For example, the enzyme lipase catalyses the hydrolysis of the ester of 2-hydroxymethyl-5-oxo-1,3-oxathiolane without hydrolysing the internal lactone. This contrasts markedly with "chemical" approaches to ester hydrolysis.

Still another advantage of this method is that the separation of the unhydrolysed enantiomer and the hydrolysed enantiomer from the reaction mixture is quite simple. The unhydrolysed enantiomer is more lipophilic than the hydrolysed enantiomer and can be efficiently recovered by simple extraction with one of a wide variety of nonpolar organic solvents or solvent mixtures, including hexane and hexane/ether. The less lipophilic, more polar hydrolysed enantiomer can then be obtained by extraction with a more polar organic solvent, for example, ethyl acetate, or by lyophilization, followed by extraction with ethanol or methanol. Alcohol should be avoided during the hydrolysis because it can denature enzymes under certain conditions.

### Enzymes and Substrates

With the proper matching of enzyme and substrate, conditions can be established for the isolation of either nucleoside enantiomer. The desired enantiomer can be isolated by treatment of the racemic mixture with an enzyme that hydrolyses the desired enantiomer (followed by extraction of the polar hydrolysate with a polar solvent) or by treatment with an enzyme that hydrolyses the undesired enantiomer (followed by removal of the undesired enantiomer with a nonpolar solvent).

Enzymes that catalyze the hydrolysis of esters include esterases, for example pig liver esterase, lipases, including porcine pancreatic lipase and Amano PS-800 lipase, subtilisin, and α-chymotrypsin.

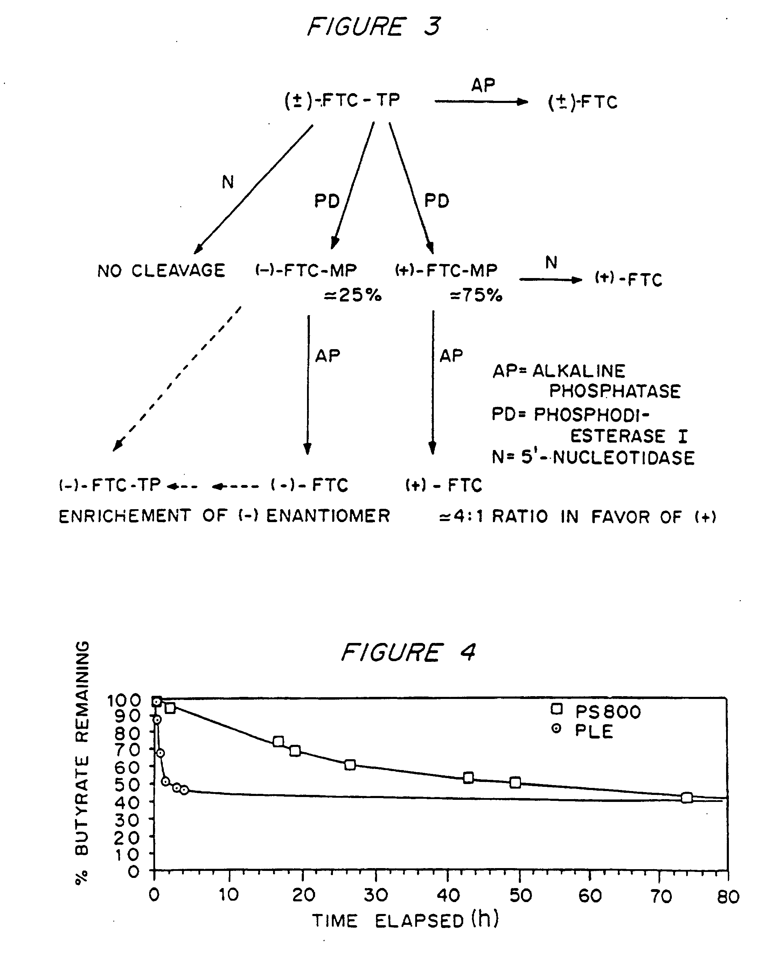

Figure 3 is a flow chart of the specificity of alkaline phosphatase and snake venom phosphodiesterase for the (+) and (-) enantiomers of FTC. As indicated, alkaline phosphatase hydrolyses the triphosphate of both of the enantiomers to FTC, and therefore is not effective as a separation means. Phosphodiesterase I preferentially hydrolyses the (+)-isomer of FTC to its monoester, which can then be exposed to 5'-nucleotidase to provide (+)-FTC.

The most effective acyl group to be used to esterify the C5'-position of the nucleoside can be determined without undue experimentation by evaluation of a number of homologs using the selected enzyme system. For example, when 1,3-oxathiolane nucleosides are esterified with butyric acid, resolutions with both pig liver esterase and Amano PS-800 proceed with high enantioselectivity (94-100% enantiomeric excess) and opposite selectivity. Pig liver esterase preferentially hydrolyses the (+)-enantiomer of FTC, and Amano PS-800® preferentially hydrolyses the (-)-enantiomer of FTC. The percent enantiomeric excess reported in Table 1 is the amount of purified butyrate ester remaining in the enzyme treated mixture (i.e., the butyrate ester of (-)-FTC in the case of PLE and the butyrate ester of (+)-FTC in the case of Amano PS-800®).

Non-limiting examples of acyl groups that can be evaluated for use with a particular nucleoside enantiomeric mixture and particular enzyme include alkyl carboxylic acids and substituted alkyl carboxylic acids, including acetic acid, propionic acid, butyric acid; and pentanoic acid. With certain enzymes, it may be preferred to use an acyl compound that is significantly electron-withdrawing to facilitate hydrolysis by weakening the ester bond. Examples of electron-withdrawing acyl groups include α-haloesters such as 2-chloropropionic acid, 2-chlorobutyric acid, and 2-chloropentanoic acid. α-Haloesters are excellent substrates for lipases.

### Resolution Conditions

The enzymatic hydrolyses are typically carried out with a catalytic amount of the enzyme in an aqueous buffer that has a pH that is close to the optimum pH for the enzyme in question. As the reaction proceeds, the pH drops as a result of liberated carboxylic acid. Aqueous base should be added to maintain the pH near the optimum value for the enzyme. The progress of the reaction can be easily determined by monitoring the change in pH and the amount of base needed to maintain pH. The hydrophobic ester (the unhydrolysed enantiomer) and the more polar alcohol (the hydrolysed enantiomer) can be sequentially and selectively extracted from the solution by the judicious choice of organic solvents. Alternatively, the material to be resolved can be passed through a column that contains the enzyme immobilized on a solid support.

Enzymatic hydrolyses performed under heterogeneous conditions can suffer from poor reproducibility. Therefore, it is preferred that the hydrolysis be performed under homogeneous conditions. Alcohol solvents are not preferred because they can denature the enzymes. Homogeneity can be achieved through the use of non-ionic surfactants, such as Triton X-100. However, addition of these surfactants not only assists in dissolving the starting material, they also enhance the aqueous solubility of the product. Therefore, although the enzymatic reaction can proceed more effectively with the addition of a non-ionic surfactant than under heterogeneous conditions, the isolation of both the recovered starting material and the product can be made more difficult. The product can be isolated with appropriate chromatographic and chemical (e.g., selective salt formation) techniques. Diacylated nucleosides can be used but are often quite lipophilic and hard to dissolve in the medium used.

### Example 1: Enantioselective Lipase-Catalysed Hydrolysis of FTC Esters.

A number of 5'-O-acyl derivatives of FTC were prepared by selective O-acylation of the N-hydrochloride salt (see Table 1 and Figure 4) of (±)-FTC. The efficiency of the hydrolysis of the derivatives by lipases was investigated. As shown in Table 1, pig liver esterase (PLE) exhibits a high level of selectivity for the hydrolysis of the ester of the (+)-enantiomer of FTC, leaving predominately the butyrate of (-)-FTC in the HPLC-analyzed mixture. In contrast, PS-800 hydrolyses the ester of the (-)-enantiomer of FTC preferentially, leaving predominately the butyrate of the (+)-FTC in the HPLC-analyzed mixture. The rate of the hydrolysis was also found to be dependent on the nature of the acyl group; the acetyl derivative was significantly slower than the butyryl derivative. It has now been discovered that the rate of the hydrolysis of the propionic acid ester of FTC is even faster than that observed for the butyrate derivative. Percent recovery and percent of enantiomeric excess were both determined using HPLC. Although the enantioselectivity is excellent when employing PLE (typically 97% e.e. or higher), additional enrichment can be accomplished by sequential enzymatic hydrolysis reactions in which the enantiomerically-enriched butyrate from a PLE-catalyzed hydrolysis is subjected to enzymatic hydrolysis by PS-800.

**Table 1**

| **Comparison of Effect of Ester on Enzyme Hydrolysis** | | |
|---|---|---|
| Substrate | % Recovery | % E.E. (s.m.) |
| **FTC Esters with PLE:** | | |
| | | (-)-FTC (butyrate) |
| acetate | 32.68 | N.D. |
| propionate | 39.87 | N.D. |
| butyrate | 48.00 | 98 |
| butyrate | 45.71 | 98.6 |

| **FTC Esters with PS800:** | | |
|---|---|---|
| | | (+)-FTC butyrate |
| acetate | 73.17 | N.D. |
| propionate | 52.67 | N.D. |
| butyrate | 58.34 | N.D. |
| valerate | 41.50 | 94 |

### Example 2: Procedure for the Preparation of (+)- and (-)-FTC via Enantioselective, Lipase-Catalyzed Hydrolysis of FTC Butyrate.

The 5'-O-butyrate of (±)-FTC (0.47 mmol, 149 mg) was dissolved in 16 mL of a solution of 4:1 pH 8 buffer:CH₃CN. The clear solution was stirred and treated with 26 mg of pig liver esterase (PLE-A). The progress of the reaction was monitored by HPLC (Figure 4). After 20 hours (52% conversion), the reaction mixture was extracted with 2 x 80 mL of CHCl₃ and 80 mL of ethyl acetate. The organic layer extracts were combined, dried over anhydrous MgSO₄, filtered, and concentrated by rotary evaporation. The resulting residue was eluted on 2 x 1000m pTLC plates using ethyl acetate as eluant (double elution) to give, after isolation, 53 mg (36% based on starting material) of FTC butyrate which was determined to have 98% enantiomeric excess (e.e.) by HPLC analysis. The enantiomerically-enriched butyrate was then treated with 1.6 mL of methanol followed by 0.38 mmol (20 mg) of sodium methoxide. The resulting mixture was stirred at room temperature, and the progress of the reaction was monitored by HPLC. The reaction was completed within 30 minutes. The solvent was removed by rotary evaporation to give a crude white solid (76 mg) that was eluted on a 1000m pTLC using 5:1 ethyl acetate:ethanol. (-)-FTC was isolated as a white solid (33 mg; 82% yield). HPLC analysis of the FTC as its 5'-O-acetate derivative showed 97% e.e.; [α](²⁰,_{D}) -120.5° (c = 0.88; abs. ethanol).

Emulsions in the work-up step can be avoided by adding HCCl₃ to the reaction mixture on completion (which also serves to denature the enzyme), stripping the solvents under vacuum, and then extracting with HCCl₃.

Similarly, 1.2 mmol (375 mg) of the 5'-O-butyrate of (±)-FTC was dissolved in 40 mL of 4:1 pH 8 buffer-CH₃CN. The clear solution was stirred and treated with 58 mg of pig liver esterase (PLE-A). The progress of the reaction was monitored by HPLC. After 90 minutes (38% conversion), the reaction mixture was added to 150 mL of CHCl₃. The layers were separated and the aqueous layer lyophilized to remove solvent. The white residue from the lyophilization was extracted with 3 x 10 mL of absolute ethanol. The extracts were filtered, combined, and concentrated *in vacuo* to yield 179 mg of crude oil. The crude material was eluted on a 45 x 30 mm column of silica gel using 3 x 75 mL of ethyl acetate followed by 5:1 ethyl acetate-ethanol. (+)-FTC was isolated as a white solid (109 mg; 37% based on starting butyrate). HPLC analysis of the (+)-FTC as its 5'-O-acetate derivative showed 97.4% e.e.; [a]O(²⁰,_{D}) +113.4° (c = 2.53; absolute ethanol)

A similar reaction was performed using 0.12 mmol (37 mg) of the 5'-O-butyrate of FTC and 7 mg of PS-800 in 4.0 mL of 4:1 pH 8 buffer:CH₃CN. The reaction was considerably slower than that with PLE-A and required 74 hours for 59% conversion. The recovered butyrate (11.4 mg; 31% of the initial amount) was found to exhibit 94% e.e. by HPLC.

### Combination of Enzymatic Resolution with Classical Resolution Methods

The process described above for resolving racemic mixtures of nucleoside enantiomers can be combined with other classical methods of enantiomeric resolution to increase the optical purity of the final product.

Classical methods of resolution include a variety of physical and chemical techniques. Often the simplest and most efficient technique is recrystallization, based on the principle that racemates are often more soluble than the corresponding individual enantiomers. Recrystallization can be performed at any stage, including on the acylated compounds or the final enantiomeric product. If successful, this simple approach represents a method of choice.

When recrystallization fails to provide material of acceptable optical purity, other methods can be evaluated. If the nucleoside is basic (for example, a cytidine) one can use chiral acids that form diastereomeric mixtures that may possess significantly different solubility properties. Nonlimiting examples of chiral acids include malic acid, mandelic acid, dibenzoyl tartaric acid, 3-bromocamphor-8-sulfonic acid, 10-camphorsulfonic acid, and di-p-toluoyltartaric acid. Similarly, acylation of the free hydroxyl group with a chiral acid derivative also results in the formation of diastereomeric mixtures whose physical properties may differ sufficiently to permit separation.

Small amounts of enantiomerically enriched nucleosides can be obtained or purified by passing the racemic mixture through an HPLC column that has been designed for chiral separations, including cyclodextrin bonded columns marketed by Rainin Corporation.

### Example 3: Separation of Racemic Mixtures of Nucleosides by HPLC.

The resolutions of the C4'-enantiomers of (±)-FTC were performed using a chiral cyclodextrin bonded (cyclobond AC-I) column obtained from Rainin Corporation (Woburn, MA). The conditions were as follows: Isocratic 0.5% methanol in water; flow rate 1 ml/min., UV detection at 262 nm. HPLC grade methanol was obtained from J.T. Baker (Phillipsburg, NJ). The racemic mixtures were injected and fractions were collected. Fractions containing each of the enantiomers were pooled, frozen, and then lyophilized. The compounds were characterized by UV spectroscopy and by their retention times on HPLC. In general, the (-)-enantiomers have lower retention times than the (+)-enantiomers (see J. Liquid Chromatography 7:353-376, 1984). The concentrations of the compounds were determined by UV spectroscopy, using a stock solution of known concentration (15 µM) prepared in water for biological evaluation. The retention times for the separated enantiomers are provided in Table 2.

**Table 2**

| **Retention Times of Enantiomers of FTC** | |
|---|---|
| Compound | R_{f} (min) |
| (-)-FTC | 8.3 |
| (+)-FTC | 8.7 |

### Example 4 Alternative Methods for Separating FTC Enantiomers using a Chiral Column

Using a Cyclobond I-Ac column (5 µm, 25 cm x 4.6 mm, Rainin Corporation, Woburn, MA, catalog no. AST-41049), with a flow rate of 0.6 ml/min of 0.5% isocratic methanol (Fisher Scientific, Inc. HPLC grade, cat no. A-452-4 in water), and UV detection at 262 nm, the FTC enantiomers exhibited retention times of 12.68 minutes ((-)-FTC) and 13.20 minutes ((+)-FTC).

Using a Chiralpak AS column (10 µm, 25 cm x 4.6 mm, J.T. Baker Inc., Phillisburg, NJ, catalog no. 7406-00, serial no. 09-29-10320) with a flow rate of 0.8 ml/min of isopropyl alcohol (HPLC grade, Fisher Scientific, Inc., cat no. A-451-4) and UV detection of 262 nm, the FTC enantiomers exhibited retention times of 5.9 minutes ((-)-FTC), and 9.8 minutes ((+)-FTC)

### IV. Ability of 2-Hydroxymethyl-5-(5-Fluorocytosin-1-yl)-1,3-Oxathiolane ("FTC") to Inhibit the Replication of HIV

It is often desirable to screen a number of racemic mixtures of nucleosides as a preliminary step to determine which warrant further resolution into enantiomerically enriched components and further evaluation of antiviral activity. The ability of nucleosides to inhibit HIV can be measured by various experimental techniques. The technique used herein, and described in detail below, measures the inhibition of viral replication in phytohemagglutinin (PHA) stimulated human peripheral blood mononuclear (PBM) cells infected with HIV-1 (strain LAV). The amount of virus produced is determined by measuring the virus-coded reverse transcriptase enzyme. The amount of enzyme produced is proportional to the amount of virus produced. Table 3 provides the EC₅₀ values (concentration of nucleoside that inhibits the replication of the virus by 50% in PBM cells, estimated 10% error factor) and IC₅₀ values (concentration of nucleoside that inhibits 50% of the growth of mitogen-stimulated uninfected human PBM cells) of a number of (±)-1,3-oxathiolane and nucleosides.

### Example 5: Anti-HIV Activity of (±)-1,3-Oxathiolane Nucleosides.

A. Three-day-old phytohemagglutinin-stimulated PBM cells (10⁶ cells/ml) from hepatitis B and HIV-1 seronegative healthy donors were infected with HIV-1 (strain LAV) at a concentration of about 100 times the 50% tissue culture infectious dose (TICD 50) per ml and cultured in the presence and absence of various concentrations of antiviral compounds.
B. Approximately one hour after infection, the medium, with the compound to be tested (2 times the final concentration in medium) or without compound, was added to the flasks (5 ml; final volume 10 ml). AZT was used as a positive control.
C. The cells were exposed to the virus (about 2 x 10⁵ dpm/ml, as determined by reverse transcriptase assay) and then placed in a CO₂ incubator. HIV-1 (strain LAV) was obtained from the Center for Disease Control, Atlanta, Georgia. The methods used for culturing the PBM cells, harvesting the virus and determining the reverse transcriptase activity were those described by McDougal et al. (J. Immun. Meth. 76, 171-183, 1985) and Spira et al. (J. Clin. Meth. 25, 97-99, 1987), except that fungizone was not included in the medium (see Schinazi, et al., Antimicrob. Agents Chemother. 32, 1784-1787 (1988); Id., 34:1061-1067 (1990)).
D. On day 6, the cells and supernatant were transferred to a 15 ml tube and centrifuged at about 900 g for 10 minutes. Five ml of supernatant were removed and the virus was concentrated by centrifugation at 40,000 rpm for 30 minutes (Beckman 70.1 Ti rotor). The solubilized virus pellet was processed for determination of the levels of reverse transcriptase. Results are expressed in dpm/ml of sampled supernatant. Virus from smaller volumes of supernatant (1 ml) can also be concentrated by centrifugation prior to solubilization and determination of reverse transcriptase levels.
The median effective (EC₅₀) concentration was determined by the median effect method (Antimicrob. Agents Chemother. 30, 491-498 (1986). Briefly, the percent inhibition of virus, as determined from measurements of reverse transcriptase, is plotted versus the micromolar concentration of compound. The EC₅₀ is the concentration of compound at which there is a 50% inhibition of viral growth.
E. Mitogen stimulated uninfected human PBM cells (3.8 x 10⁵ cells/ml) were cultured in the presence and absence of drug under similar conditions as those used for the antiviral assay described above. The cells were counted after 6 days using a hemacytometer and the trypan blue exclusion method, as described by Schinazi et al., Antimicrobial Agents and Chemotherapy, 22(3), 499 (1982). The IC₅₀ is the concentration of compound which inhibits 50% of normal cell growth.

**Table 3**

| **EC**_{**50**} **and IC**_{**50**} **of Various Analogues of 1,3-Oxathiolane Nucleosides in Human PBM Cells** | | | | |
|---|---|---|---|---|
| Code | X or Y | R | Antiviral Cytotoxicity | |
| | | | EC₅₀, µM | IC₅₀, µM |
| DLS-009 | X = O | H | >100 | >100 |
| DLS-010 | X = O | Me | 64.4 | >100 |
| DLS-027 | X = O | F | >100 | >100 |
| DLS-028 | X = O | Cl | 60.8 | >100 |
| DLS-044 | X = O | Br | >100 | >100 |
| DLS-029 | X = O | I | >100 | >100 |
| DLS-020 | Y = NH₂ | H | 0.02 | >100 |
| DLS-011 | Y = NH₂ | Me | >10 | >100 |
| DLS-022 | Y = NH₂ | F | 0.01 | >100 |
| DLS-023 | Y = NH₂ | Cl | 38.7 | >100 |
| DLS-021 | Y = NH₂ | Br | 77.4 | >100 |
| DLS-026 | Y = NH₂ | I | 0.72 | >100 |
| DLS-058(-) | Y = NH₂ | F | 0.008 | >100 |
| DLS-059(+) | Y = NH₂ | F | 0.84 | >100 |
| DLS-053 | Y = NH₂ | CF₃ | 60.7 | >100 |

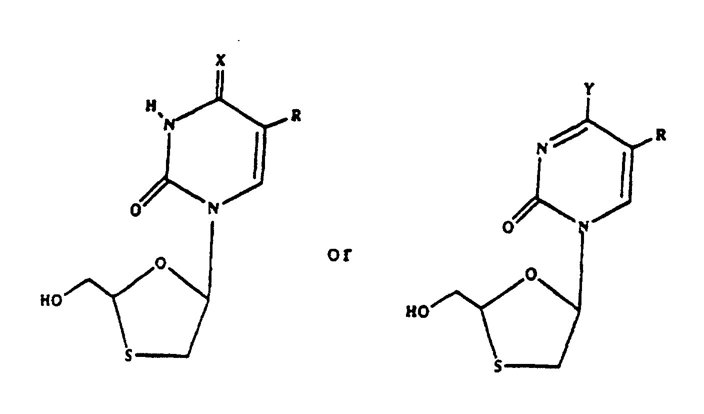

As indicated in Table 3, in general, the substituted cytosine 1,3-oxathiolane nucleosides are more active than the corresponding uracil nucleosides. The error in EC₅₀ and IC₅₀ measurements are estimated at ±10%.

One of the compounds, (±)-FTC, (referred to as "DLS-022", compound 8) not only exhibits exceptional activity (approximately 10 nM in PBM cells), but also quite low toxicity (>100 µM in PBM, Vero and CEM cells).

The IC₅₀ of (±)-FTC was over 100 µM, indicating that the compound was not toxic in uninfected PBM cells evaluated up to 100 µM.

### Example 6: Antiviral Activity of the Enantiomers of FTC Resolved by HPLC.

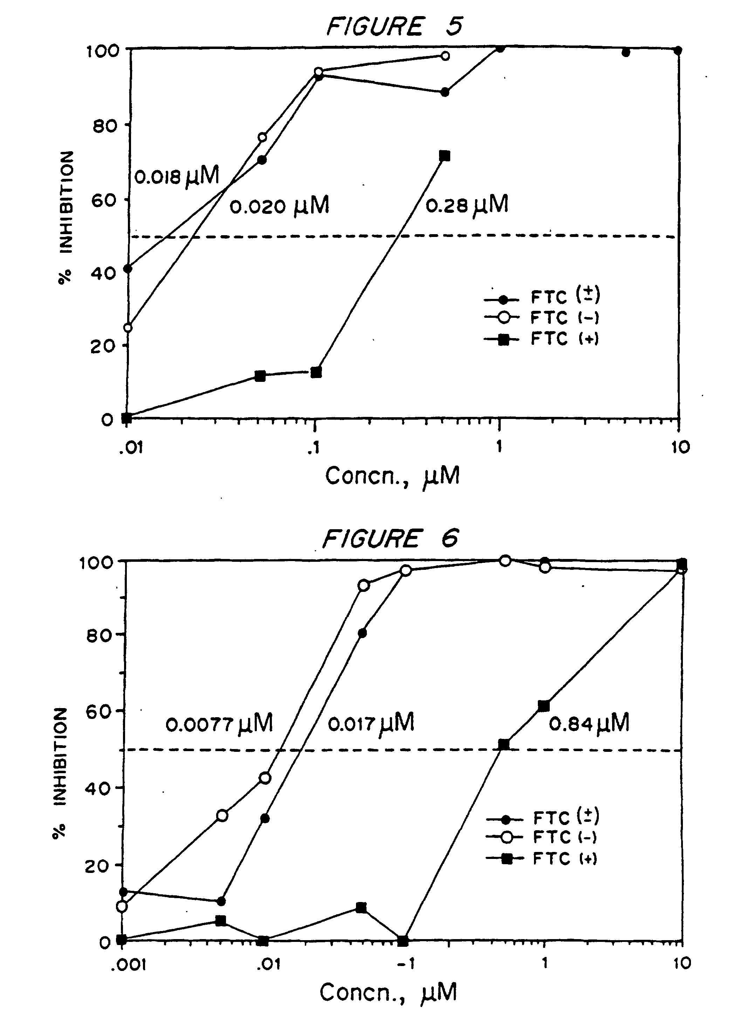

The enantiomers of FTC were isolated by the method of Example 3, and the antiviral activity evaluated by the method of Example 5. The results are provided in Table 4, and illustrated in Figure 5.

**Table 4**

| **Antiviral Activity of the (+) and (-) Enantiomers of FTC** | | | | |
|---|---|---|---|---|
| Treatment Concn., µM | | DPM/ml | % Inhibition (Corrected) | EC₅₀:µM |
| FTC (±) | 0.0001 | 73,755 | 26.6 | 0.018 |
| | 0.005 | 83,005 | 16.3 | |
| | 0.01 | 60,465 | 41.3 | |
| | 0.05 | 34,120 | 70.4 | |
| | 0.1 | 14,160 | 92.4 | |
| | 0.5 | 18,095 | 88.1 | |
| | 1 | 7,555 | 99.7 | |
| | 5 | 7,940 | 99.3 | |
| | 10 | 5,810 | 101.7 | |
| | | | | |
| FTC (-) | 0.001 | 76,275 | 23.8 | 0.02 |
| | 0.005 | 58,590 | 43.3 | |
| | 0.01 | 75,350 | 24.8 | |
| | 0.05 | 28,890 | 76.2 | |
| | 0.1 | 13,175 | 93.5 | |
| | 0.5 | 9,485 | 97.6 | |
| | | | | |
| FTC (+) | 0.001 | 94,340 | 3.8 | 0.28 |
| | 0.005 | 107,430 | -10.6 | |
| | 0.01 | 99,465 | -1.8 | |
| | 0.05 | 87,120 | 11.8 | |
| | 0.1 | 86,340 | 12.7 | |
| | 0.5 | 33,225 | 71.4 | |

As indicated in Table 4, in this experiment the (-)-enantiomer of FTC appears to be approximately one order of magnitude more potent than the (+)-FTC enantiomer, and has approximately the same anti-HIV activity as the racemic mixture. Neither the enantiomers nor the racemic mixture is toxic up to 100 µM as measured by the Trypan Blue exclusion method in human PBM cells.

### Example 7: Antiviral Activity of FTC Enantiomers Resolved by Method of Example 2.

The enantiomers of (±)-FTC were also resolved by the method of Example 2, and the antiviral activity evaluated by the method of Example 5. The results are illustrated in Figure 6. As indicated in Figure 6, the EC₅₀ of the racemic mixture of FTC was 0.017 µM, the EC₅₀ of (-)-FTC at 0.0077 µM, and the EC₅₀ of (+)-FTC at 0.84 µM.

### Example 8: Uptake of (±)-FTC into Human PBM Cells

Studies were undertaken using radiolabeled FTC to follow the intracellular profiles of the parent drug and metabolites detected within the cell. All studies were conducted in duplicate. Human peripheral blood mononuclear cells (PBM cells) were suspended in RPMI 1640 medium containing 10% fetal calf serum and antibiotics (2 x 10⁶ cells/ml), 10 ml per timepoint) and incubated with addition of 10 µM FTC (specific activity about 700 dpm/pmol). Cells were exposed to the drug for 2, 6, 12, and 24 hours. At these timepoints, the medium was removed and the cells were washed two times with cold Hank's balanced salt solution. Extraction was performed with addition of 0.2 ml of 60% cold methanol/water and stored overnight at -70°C. The following morning, the suspensions were centrifuged and extractions were repeated two times for 0.5 hours at -70°C. The total supernatants (0.6 ml) were lyophilized to dryness. The residues were resuspended in 250 µl of water and aliquots of between 50 and 100 µl were analyzed by HPLC. Quantitation of intracellular parent drug and metabolic derivatives were conducted by HPLC. Because of the potential acid lability of some compounds, a buffer system close to physiological pH was used for the separation of the metabolites.

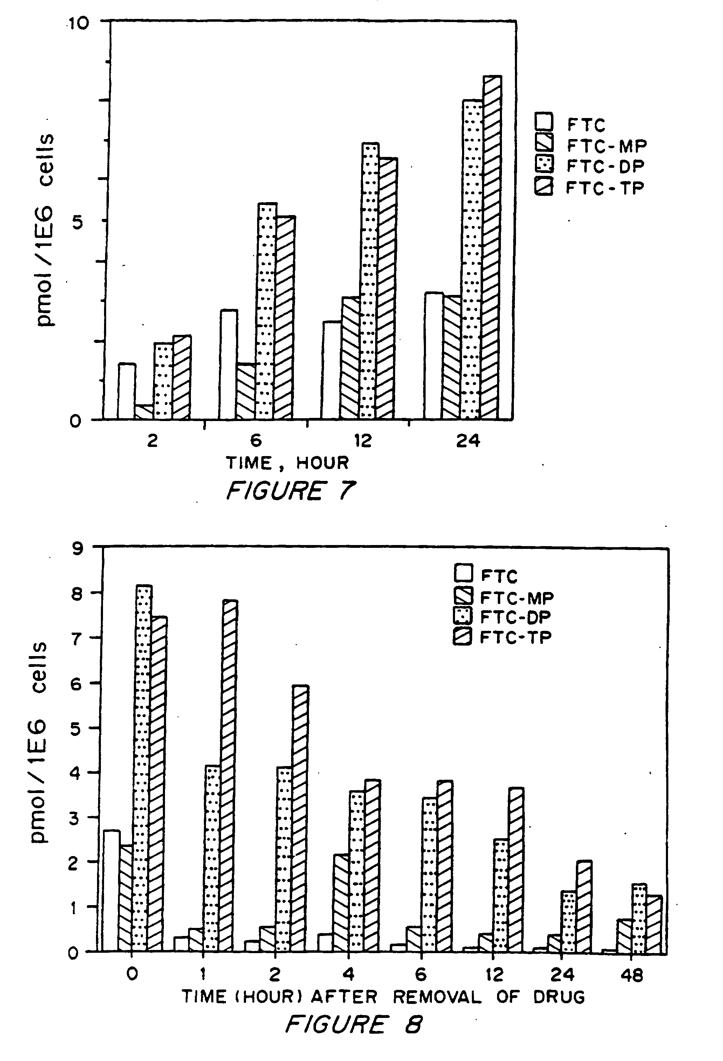

Figure 7 is a graph of the presence (uptake) of tritiated (±)-FTC in human PBM cells (average of two determinations) in time (hours) versus pmol/10⁶ cells. The uptake studies indicate that radiolabeled FTC is readily taken up in human lymphocytes, that produce very large amounts of the 5'-triphosphate derivative of FTC.

### Example 9 Antiretroviral Activity of FTC in Various Cell Lines

The antiretroviral activity of FTC was measured in a number of cell lines using procedures similar, but not identical, to that set out in Example 5. Cell lines were obtained from either human donors, AIDS Research and Reference Reagent Program, NIH, Rockville, Maryland, ATCC, or the Red Cross. The CEM thymidine kinase deficient cells were prepared by sequential passage of CEM cells in the presence of 5-bromo-2'-deoxyuridine. The results are provided in Table 5.

**Table 5**

| **Antiretroviral Activity of FTC In Different Cell Systems** | |
|---|---|
| **Cell system (Virus strain)** | **EC**_{**50**} **(µM) (±)-FTC** |
| **HIV-1** | |
| PBMC (LAV-1) | 0.027 |
| MT2 (HTLV_{IIIB}) | 0.89 |
| CEM (LAV-1) | 0.08 |
| CEM-TK⁽⁻⁾ (LAV-1) | 0.026 |
| CEM (HTLV_{IIIB}) NIH | 0.09 |

| **HIV-2** | |
|---|---|
| PBMC (ROD2) | 0.0038 (±)-FTC |
| | 0.0007 (-)-FTC |
| | 0.026 (+)-FTC |

| **SIV** | |
|---|---|
| AA-2 (SIV251) | 4.6 |
| C-8166 (SIV251) | <8.0 |

| **FIV** | |
|---|---|
| CrFK (61E) | ≤1 |

### Example 10: Egress of (±)-FTC from Human PBM Cells.

Studies were performed using radiolabeled FTC to follow the intracellular profiles of the parent drug and metabolites detected within the cell after incubation in media with drug for 24 hours, and then removal of drug. This study measures the time needed for intracellular levels of triphosphates to decline. Studies were conducted in duplicate. Uninfected cells (2 x 10⁶ ml) were suspended in the appropriate medium supplemented with serum (10 ml per timepoint) and incubated at 37°C in a 5% CO₂ incubator. The radiolabeled FTC concentration was 10 µM. After pulsing the cells with the labeled compound for 24 hours, the cells were thoroughly washed and then replenished with fresh medium without the antiviral drugs (0 hr). At 0, 2, 4, 6, 12, 24, and 48 hours (second incubation time), the cells were removed, and immediately extracted with 60% cold methanol/water. The extract was obtained by centrifugation and removal of the cell pellet. The extracts were lyophilized and then stored at -70°C. Prior to analysis, the material was resuspended in 250 microliters of HPLC buffer and immediately analyzed. Quantitation of intracellular parent drug and metabolic derivatives was conducted by HPLC, using either a Micromeritics or Hewlett-Packard model 1090 PHLC system with an anion exchange Partisil 10 SAX column (Whatman, Inc.), at a flow rate of 1 ml/min, 1 kpsi pressure, with UV detection at 262 nm. The mobile phase consisted of deionized water (A), 2 mM NaH₂PO₄/16 mM NaOAc (pH = 6.6) (B), 15 mM NaH₂PO₄/120.2 mM NaOAc (pH = 6.6) (C), and 100 mM NaH₂PO₄/800 mM NaOAc (pH = 6.6) (D).

Separation method: isocratic for 5 minutes with A, followed by a 15 minute linear gradient to 100% B, followed by a 20 minute linear gradient to 100% C, followed by 10 minute linear gradient to 100% D, followed by 30 minutes isocratic with 100% D.

| Retention times (minutes) in Human Cells: | | | | |
|---|---|---|---|---|
| Compound | Unchanged | Monophosphate | Diphosphate | Triphosphate |
| (±)-FTC | 5.0 | 39.0 | 55.0 | 68.0 |

Figure 8 is a graph of the egress of radiolabeled (±)-FTC from human PBM cells, measured in hours after drug removal versus concentration (pmol/10⁶ cells). As indicated in the Figure, FTC-triphosphate has an intracellular half-life of approximately 12 hours and can be easily detected intracellularly at concentrations of 1-5 µM 48 hours after the removal of the extracellular drug, which is well above the EC₅₀ for the compound. Further, the affinity (K¹) for (±)-FTC triphosphate using HIV RT is 0.2 µM, which is below the 48 hour concentration level.

### Example 11 Anti-HIV Activity of Pharmaceutically Acceptable Derivatives of (±)-FTC

a. A number of pharmaceutically acceptable derivatives of (±)-FTC prepared by derivatizing the 5' and N⁴ positions were evaluated for anti-HIV activity in PBM cells using a procedure similar to that described in Example 5. The results are as follows. The 5'-o-butyrate ester of (±)-FTC exhibited an EC₅₀ of 0.0017. The N⁴-acetyl derivative of (±)-FTC exhibited an EC₅₀ of 0.0028. The 5'-o-butyrate, N⁴-ester of (±)-FTC exhibited an EC₅₀ = 0.0058.
b. The anti-HIV activity of the 5'-O-butyrate ester of (±)-FTC in the MT4 system (EC₅₀) was 0.04 µM. In the same assay, the unacylated (±)-FTC exhibited an IC₅₀ of 0.52 µM. The IC50 for AZT in this system was 0.09 µM.

### V. Ability of FTC to Inhibit the Replication of HBV

### Example 12 Evaluation of activity of (+) and (-)-Enantiomers of FTC in 2.2.15 Cell Cultures

The ability of the enantiomers of FTC to inhibit the growth of virus in 2.2.15 cell cultures (HepG2 cells transformed with hepatitis virion) is described in detail below.

A summary and description of the assay for antiviral effects in this culture system and the analysis of HBV DNA has been described (Korba and Milman, 1991, Antiviral Res., 15:217). The antiviral evaluations were performed on two separate passages of cells. All wells, in all plates, were seeded at the same density and at the same time.

### ASSAY PARAMETERS

Due to the inherent variations in the levels of both intracellular and extracellular HBV DNA, only depressions greater than 3.5-fold (for HBV virion DNA) or 3.0-fold (for HBV DNA replication intermediates) from the average levels for these HBV DNA forms in untreated cells are considered to be statistically significant [P<0.05]. The levels of integrated HBV DNA in each cellular DNA preparation (which remain constant on a per cell basis in these experiments) were used to calculate the levels of intracellular HBV DNA forms, thereby ensuring that equal amounts of cellular DNA were compared between separate samples.

Typical values for extracellular HBV virion DNA in untreated cells ranged from 50 to 150 pg/ml culture medium (average of approximately 76 pg/ml). Intracellular HBV DNA replication intermediates in untreated cells ranged from 50 to 100 pg/µg cell DNA (average approximately 74 pg/µg cell DNA). In general, depressions in the levels of intracellular HBV DNA due to treatment with antiviral compounds are less pronounced, and occur more slowly, than depressions in the levels of HBV virion DNA (Korba and Milman, 1991, Antiviral Res., 15:217).

The manner in which the hybridization analyses were performed for these experiments resulted in an equivalence of approximately 1.0 pg of intracellular HBV DNA to 2-3 genomic copies per cell and 1.0 pg/ml of extracellular HBV DNA to 3 x 10⁵ viral particles/ml.

### TOXICITY ANALYSIS

Toxicity analyses were performed to assess whether any observed antiviral effects were due to a general effect on cell viability. The method used herein was the measurement of the uptake of neutral red dye, a standard and widely used assay for cell viability in a variety of virus-host systems, including HSV and HIV. Toxicity analyses were performed in 96-well flat bottomed tissue culture plates. Cells for the toxicity analyses were cultured and treated with test compounds with the same schedule as described for the antiviral evaluations below. Each compound was tested at 4 concentrations, each in triplicate cultures (wells "A", "B", and "C"). Uptake of neutral red dye was used to determine the relative level of toxicity. The absorbance of internalized dye at 510 nm (Aₛᵢₙ) was used for the quantitative analysis. Values are presented as a percentage of the average Aₛᵢₙ values in 9 separate cultures of untreated cells maintained on the same 96-well plate as the test compounds. Dye uptake in the 9 control cultures on plate 5 ranged from 91.6% to 110.4%, and on plate 6 from 96.6% to 109%. The results are provided in Table 6.

**Table 6**

| **Toxicity Analysis of Test Compounds in 2.2.15 Cells** | | | | | |
|---|---|---|---|---|---|
| PLATE | COMPOUND | CONC. (µM) | DYE UPTAKE (% OF CONTROL) | | |
| | | | WELL A | WELL B | WELL C |
| 5 | DMSO | 10.0∗ | 0.7 | 1.6 | 0.9 |
| | | 3.3 | 55.9 | 68.7 | 61.7 |
| | | 1.0 | 91.2 | 96.4 | 106.8 |
| | | 0.3 | 98.7 | 102.9 | 93.5 |
| | | | | | |
| 6 | (-)-FTC | 300 | 53.0 | 51.1 | 51.5 |
| | | 100 | 64.1 | 66.6 | 77.6 |
| | | 30 | 98.7 | 94.3 | 96.4 |
| | | 10 | 94.3 | 94.9 | 92.2 |
| | | | | | |
| 6 | (+)-FTC | 300 | 43.4 | 56.7 | 58.5 |
| | | 100 | 77.7 | 66.3 | 72.1 |
| | | 30 | 81.1 | 88.3 | 88.1 |
| | | 10 | 90.9 | 99.4 | 90.5 |

| | | | | | |
|---|---|---|---|---|---|
| ∗ For DMSO, concentrations are presented as percent of original stock solution. | | | | | |

### TOXICITY EVALUATION

As indicated in Table 6, no significant toxicity (greater than 50% depression of the dye uptake levels observed in untreated cells) was observed for the test compounds at the concentrations used for the antiviral evaluations. Both test compounds, (-)-FTC and (+)-FTC, appeared to be toxic at the highest concentration used for the toxicity tests (330 µM).

### ANTIVIRAL EVALUATIONS

### CONTROLS

Within normal variations, levels of HBV virion DNA and intracellular HBV replication intermediates [HBV RI] remained constant in the untreated cells over the challenge period. DMSO, at a concentration of 1%, did not affect the levels of HBV replication in 2.2.15 cell cultures.

### TEST COMPOUNDS

As indicated in Table 7, both (-)-FTC and (+)-FTC significantly inhibited the replication of HBV at the tested levels. As indicated in Table 8, (-)-FTC still significantly inhibits the synthesis of HBV virion DNA and intracellular HBV DNA at concentrations of 4, 1, and 0.25 µM.

**Table 7**

| **Effect of Test compounds on HBV Production In 2.2.15 Cell Cultures** | | | | | | |
|---|---|---|---|---|---|---|
| | | HBV Virion DNA* (pg/ml Culture Medium) | | | Intracellular HBV DNA (pg/ug Cell DNA) | |
| WELL | TREATMENT | DAY 0 | DAY 4 | DAY 9 | MONO. | RI |
| 7A | Untreated Cells | 59 | 75 | 94 | 2.7 | 93 |
| 7B | Untreated Cells | 47 | 64 | 88 | 2.5 | 93 |
| 8A | Untreated Cells | 65 | 100 | 71 | 2.2 | 97 |
| 8B | Untreated Cells | 77 | 65 | 110 | 2.4 | 62 |
| | | | | | | |
| 7K | DMSO @ 1.00% | 100 | 50 | 48 | 1.9 | 95 |
| 7L | DMSO @ 1.00% | 48 | 96 | 54 | 2.8 | 98 |
| 8K | DMSO @ 1.00% | 93 | 63 | 68 | 2.2 | 86 |
| 8L | DMSO @ 1.00% | 66 | 57 | 59 | 1.6 | 97 |
| | | | | | | |
| 9U | (-)-FTC @ 10 µM | 120 | 36 | 1 | 1.1 | 14 |
| 9V | " @ 10 µM | 89 | 48 | 1 | 1.5 | 19 |
| 10U | " @ 10 µM | 58 | 41 | 0.1 | 1.9 | 13 |
| 10V | " @ 10 µM | 110 | 32 | 0.1 | 1.2 | 16 |
| | | | | | | |
| 9W | (+)-FTC @ 10 µM | 88 | 42 | 0.1 | 0.8 | 14 |
| 9X | " @ 10 µM | 58 | 57 | 0.2 | 0.4 | 19 |
| 10w | " @ 10 µM | 69 | 55 | 0.1 | 0.7 | 17 |
| 10X | " @ 10 µM | 45 | 39 | 0.1 | 0.4 | 15 |

| | | | | | | |
|---|---|---|---|---|---|---|
| * Sensitivity cutoff for HBV virion DNA was 0.1 pg/ml. @ Intracellular HBV DNA was analyzed 24 hours following the 9th day of treatment. The levels of integrated HBV DNA in each cell DNA preparation were used to calculate the levels of episomal 3.2Kb HBV genomes (MONO.) and HBV DNA replication intermediates (RI). | | | | | | |

**Table 8**

| **Effect of Test Compounds on HBV Production in 2.2.15 Cell Cultures** | | | | | | |
|---|---|---|---|---|---|---|
| | | HBV VIRION DNA* (pq/ml CULTURE MEDIUM) | | | INTRACELLULAR HBV DNA* (pg/µg CELL DNA) | |
| WELL | TREATMENT | DAY O | DAY 4 | DAY 9 | MONO. | RI |
| 31A | untreated cells | 64 | 54 | 65 | 2.8 | 65 |
| 31B | " | 51 | 54 | 77 | 2.0 | 53 |
| 32A | " | 100 | 76 | 56 | 3.5 | 81 |
| 32B | " | 53 | 97 | 83 | 3.1 | 68 |
| | | | | | | |
| 35A | (-)-FTC @ 4 µM | 74 | 27 | >0.1 | 1.4 | 1 |
| 35B | " | 87 | 28 | >0.1 | 0.5 | 1 |
| 36A | " | 120 | 20 | 1 | 0.9 | 1 |
| 36B | " | 59 | 16 | 0.2 | 0.2 | 2 |
| | | | | | | |
| 35C | (-)-FTC @ 1 µM | 70 | 13 | >0.1 | 1.7 | 2 |
| 35D | " | 62 | 15 | >0.1 | 1.2 | 3 |
| 36C | " | 60 | 22 | 1 | 1.4 | 2 |
| 36D | " | 89 | 28 | 0.3 | 1.5 | 4 |
| | | | | | | |
| 35E | (-)-FTC @ 0.25 µM | 84 | 15 | >0.1 | 1.5 | 4 |
| 35F | " | 89 | 16 | 4 | 2.2 | 4 |
| 36E | " | 66 | 13 | 1 | 1.8 | 8 |
| 36F | " | 49 | 19 | 0.1 | 0.3 | 9 |

| | | | | | | |
|---|---|---|---|---|---|---|
| * Sensitivity cutoff for HBV virion DNA was 0.1 pg/ml. + Analysis of intracellular HBV DNA was 24 hours following the 9th day of treatment. The levels of integrated HBV DNA in each cell DNA preparation were used to calculate the levels of episomal 3.2 kb HBV genomes (MONO.) and HBV DNA replication intermediates (RI). | | | | | | |

### Example 13: Uptake of (±)-FTC into Human Liver Cells; HVB Activity of FTC.

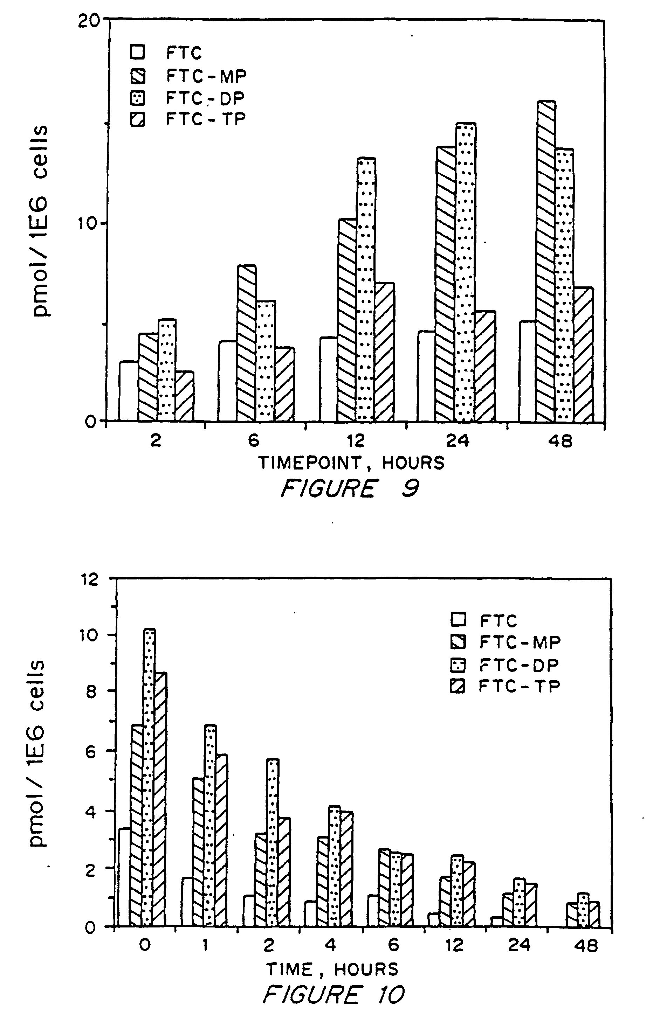

The procedure of Example 8 was repeated with human liver cells (HepG2 cells, available from the ATCC) to determine the uptake and metabolism of FTC in these cells. As shown in Figure 9, (±)-FTC is taken up by HepG2 cells in large amounts. These human liver cells metabolize a large percentage of the (±)-FTC to (±)-FTC triphosphate.

This data, in conjunction with other data provided herein, indicate that (±)-FTC, as well as its (-) and (+) enantiomers, are phosphorylated in liver cells. These cells can be transformed with hepatitis B virus.

### Example 14 Egress of FTC in Human HepG2 cells

Figure 10 illustrates the egress of [³H]-(±)-FTC and its phosphorylated derivatives in human HepG2 in pmol/10⁶ cells over time cells after pulsing cells with 10 µM [³H]-(±)-FTC (700 DPM/pmole) for 24 hours, and evaluating the concentration of compound 24 hours after removal.

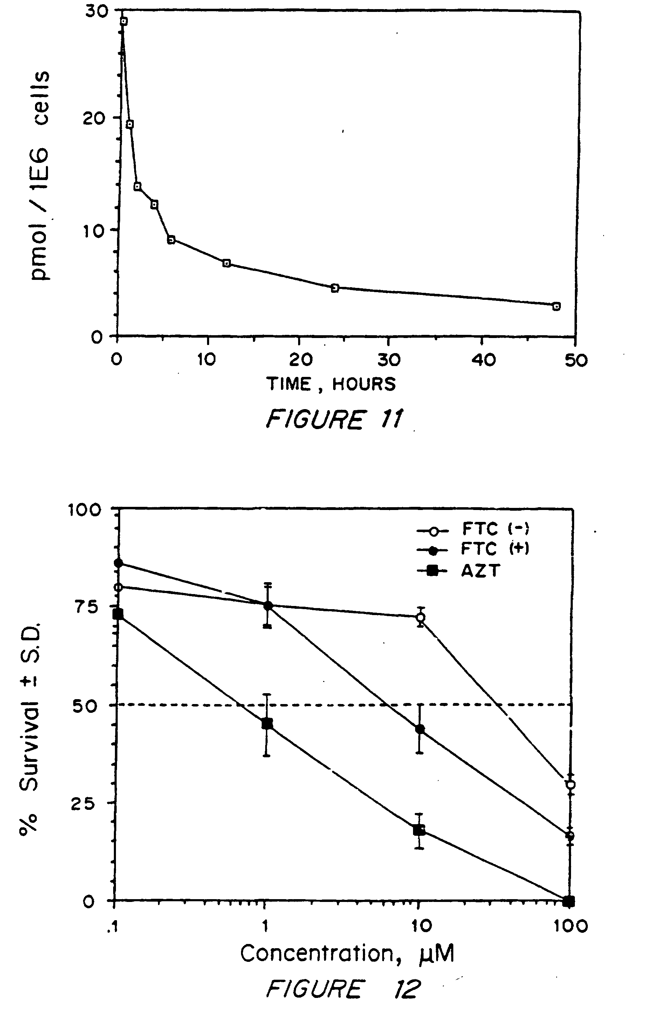

Figure 11 illustrates the decrease in the combined concentration of [³H]-(±)-FTC and its phosphorylated derivatives from human HepG2 cells after incubation with 10 µM [³H]-(±)-FTC (700 DPM/pmole) for 24 hours, in pmol/10⁶ cells over time.

As illustrated, even at 48 hours, over 1 µM of active compound (which is significantly higher than the EC₅₀ for the compound) is still present in the cells.

### V. Toxicity in Granulocyte-Macrophage Precursor Cells

### Example 15 Effect of FTC on Colony Formation of Granulocyte-Macrophage Precursor Cells

Figure 12 is a graph of the effect of the (-) and (+) enantiomers of FTC on colony formation of granulocytes-macrophage precursor cells, as measured in percent survival versus concentration in µM ((-)-FTC, open circle; (+)-FTC, darkened circle; AZT, darkened square. As indicated, the (-)-enantiomer of FTC appears to be less toxic, i.e., have a higher IC₅₀, than either the (+)-enantiomer or AZT in this cell line.

### VI. Pharmacokinetics of FTC

### Example 16 Metabolism of FTC on Administration to Rats

(±)-FTC was administered intravenously at dosages of 10, 50 and 100 mg/kg to rats, and the area under the plasma drug concentration versus time (AUC), total clearance (CL_{T}), steady-state volume of distribution (V_{SS}), mean residence time (MRT) and half-life (t_{1/2}), evaluated. The results are provided in Table 9.

**Table 9**

| **Pharmacokinetic Parameters of FTC After Intravenous Administration of 10, 50, 100 mg/kg to Rats****∗** | | | | | |
|---|---|---|---|---|---|
| Dose mg/kg | AUC mg h/L | CL_{T} L/h/kg | V_{SS} L/kg | MRT h | t_{1/2} h |
| 10 | 9.65 | 0.988 | 0.758 | 0.768 | 0.757 |
| 50 | 57.11 | 0.874 | 0.699 | 0.800 | 0.815 |
| 100 | 120.72 | 0.830 | 0.663 | 0.798 | 0.969 |

| | | | | | |
|---|---|---|---|---|---|
| ∗AUC = area under the plasma drug concentration versus time curve; CL = total clearance; V_{SS} = steady-state volume of distribution; MRT = mean residence time; and t_{1/2}= half-life. | | | | | |

### Example 17 Pharmacokinetic Parameters for FTC after Intravenous and Oral Administration of FTC

Model-independent pharmacokinetic parameters were derived for (±)-FTC by administration (intravenous (I.V.) and oral (P.O.)) of 33.3 mg/kg to rhesus monkeys. The results are provided in Table 10. Importantly, the mean bioavailability of the compound in monkeys was 73% (±6%).

**Table 10**

| **Model-Independent Pharmacokinetic Parameters Derived for FTC After Intravenous (I.V.) or Oral (P.O.) Administration of 33.3 mg/kg to Rhesus Monkeys*** | | | | | | | |
|---|---|---|---|---|---|---|---|
| Monkey | AUC mg h/L | CL_{T} L/h/kg | V_{SS} L/kg | MRT h | t_{1/2} h | Kₐ h-¹ | F % |
| I.V. | | | | | | | |
| RUh | 19.14 | 1.74 | 2.71 | 1.56 | 1.28 | | |
| RMi | 26.31 | 1.26 | 1.97 | 1.56 | 1.22 | | |
| RJd | 22.51 | 1.48 | 2.00 | 1.36 | 1.47 | | |
| | | | | | | | |
| Mean | 22.65 | 1.49 | 2.23 | 1.49 | 1.32 | | |
| ± S.D. | 3.59 | 0.24 | 0.42 | 0.12 | 0.13 | | |
| | | | | | | | |

| P.O. | | | | | | | |
|---|---|---|---|---|---|---|---|
| RUh | 13.21 | | | 2.07 | 1.58 | 0.48 | 71 |
| RMi | 21.11 | | | 2.32 | 1.08 | 0.43 | 80 |
| RJd | 15.29 | | | 3.23 | 1.47 | 0.31 | 68 |
| | | | | | | | |
| Mean | 16.54 | | | 2.54 | 1.38 | 0.41 | 73.00 (±6) |
| | | | | | | | |
| ± S.D. | 4.09 | | | 0.61 | 0.26 | 0.09 | 6.24 |

| | | | | | | | |
|---|---|---|---|---|---|---|---|
| *AUC = area under the plasma drug concentration versus time curve; CL = total clearance; V_{SS} = steady-state volume of distribution; MRT = mean residence time; and t^{1/2} = half-life; F = bioavailability; and Kₐ = first order absorption rate constant. | | | | | | | |

**Table 11**

| **CSF/Serum Ratio of PTC and Its Deaminated Metabolite 1 Hour After Treatment** | | | |
|---|---|---|---|
| Monkey | Route | FTC | Metabolite (FTU) |
| RUh | I.V. | 0.076 | 0.024 |
| RMi | I.V. | 0.062 | 0.032 |
| RJd | I.V. | 0.162 | 0.052 |
| | | | |
| Mean | | 0.100 | 0.036 |
| + S.D. | | 0.054 | 0.014 |
| | | | |
| RUh | P.O. | 0.048 | 0.026 |
| RMi | P.O. | 0.039 | 0.037 |
| RJd | P.O. | 0.117 | 0.055 |
| | | | |
| Mean | | 0.068 | 0.039 |
| ± S.D. | | 0.043 | 0.015 |

### Example 18 CSF/Serum Ratio of FTC and its Metabolites in Rhesus Monkeys

The ability of (±)-FTC to cross the blood-brain barrier was evaluated by administering 33.3 mg/kg of the active compound to rhesus monkeys, and measuring the amount of (±)-FTC in the cerebral spinal fluid (CSF) and blood serum one hour after administration. The results are provided in Table 11. The data indicates that a significant amount of active compound passes through the blood-brain barrier in this mammal.

## Claims

1. A process for obtaining (-)-β-L-2-hydroxymethyl-5-(5-fluorocytosin-1-yl)-1,3-oxathiolane, comprising resolving a mixture of (-) and (+)-cis-2-hydroxymethyl-5-(5-fluoro-cytosin-1-yl)-1,3-oxathiolane by exposing the mixture to cytidine-deoxycytidine deaminase.

2. The process of claim 1, further comprising exposing the enantiomerically enriched mixture to a second enzyme to further resolve the (-)-β-L-2-hydroxymethyl-5-(5-fluorocytosin-1-yl)-1,3-oxathiolane.

3. The process of claim 1 or claim 2, wherein the cis-2-hydroxymethyl-5-(5-fluoro-cytosin-1-yl)-1,3-oxathiolane is acylated at the C5'-hydroxyl position prior to the resolution step.

4. The process of claim 3, wherein the cis-2-hydroxymethyl-5-(5-fluoro-cytosin-1-yl)-1,3-oxathiolane is acylated with a compound selected from the group consisting of alkyl carboxylic acids and substituted alkyl carboxylic acids.

5. The process of claim 4, wherein the alkyl carboxylic acid is selected from the group consisting of acetic acid, propionic acid, butyric acid, pentanoic acid, 2-chloropropionic acid, 2-chlorobutyric acid and 2-chloropentanoic acid.

6. The process of any one of claims 2 to 5, wherein the second enzyme is selected from the group consisting of an esterase, a lipase, subtilisin, or α-chymotrypsin, which preferentially catalyze a reaction in one of the enantiomers.

7. The process of claim 6, wherein the esterase is pig liver esterase.

8. The process of claim 6, wherein the lipase is selected from the group consisting of porcine pancreatic lipase and Amano PS-800 lipase.

9. The process of any one of claims 1 to 8, wherein the mixture is mixed with the enzyme in a solution.

10. The process of any one of claims 1 to 8, wherein the mixture is passed through a column that includes the enzyme immobilized on a support.

11. The process of any one of claims 1 to 8, wherein the enzymatic reaction takes place in the presence of a non-ionic surfactant.

12. The process of claim 11, wherein the non-ionic surfactant is Triton X-100.

13. The process of any one of claims 1 to 12, further comprising recrystallizing the enantiomerically enriched mixture.

14. The process of any one of claims 1 to 13, further comprising treating the enantiomerically enriched mixture with a chiral acid selected from the group consisting of malic acid, mandelic acid, 10-camphorsulfonic acid, 3-bromocamphor-8-sulfonic acid, di-benzoyl tartaric acid and di-p-toluoyltartaric acid.

15. The process of any one of claims 1 to 14, comprising further resolving the enantiomerically enriched mixture using a chiral column.

## Patentansprüche

1. Verfahren zum Erhalten von (-)-β-L-2-Hydroxymethyl-5-(5-fluorcytosin-1-yl)-1,3-oxathiolan, welches das Trennen eines Gemisches von (-) und (+)-cis-2-Hydroxymethyl-5-(5-fluorcytosin-1-yl)-1,3-oxathiolan durch Aussetzen des Gemisches einer Cytidin-Desoxycytidin-Deaminase umfaßt.

2. Verfahren nach Anspruch 1, welches weiter das Aussetzen des enantiomerisch angereicherten Gemisches einem zweiten Enzym umfaßt, um das (-)-β-L-2-Hydroxymethyl-5-(5-fluorcytosin-1-yl)-1,3-oxathiolan weiter zu trennen.

3. Verfahren nach Anspruch 1 oder Anspruch 2, wobei das cis-2-Hydroxymethyl-5-(5-fluorcytosin-1-yl)-1,3-oxathiolan vor dem Trennungsschritt an der C5'-Hydroxylstellung acetyliert wird.

4. Verfahren nach Anspruch 3, wobei das cis-2-Hydroxymethyl-5-(5-fluorcytosin-1-yl)-1,3-oxathiolan mit einer Verbindung, ausgewählt aus der Gruppe, bestehend aus Alkylcarbonsäuren und substituierten Alkylcarbonsäuren, acetyliert wird.

5. Verfahren nach Anspruch 4, wobei die Alkylcarbonsäure aus der Gruppe, bestehend aus Essigsäure, Propionsäure, Buttersäure, Valeriansäure, 2-Chlorpropionsäure, 2-Chlorbuttersäure und 2-Chlorvaleriansäure, ausgewählt ist.

6. Verfahren nach einem der Ansprüche 2 bis 5, wobei das zweite Enzym aus der Gruppe, bestehend aus einer Esterase, einer Lipase, Subtilisin oder α-Chymotrypsin, ausgewählt ist, welches vorzugsweise eine Reaktion in eines der Enantiomere katalysiert.

7. Verfahren nach Anspruch 6, wobei die Esterase Schweinerleberesterase ist.

8. Verfahren nach Anspruch 6, wobei die Lipase aus der Gruppe, bestehend aus Pankreaslipase aus Schwein und Amano PS-800 Lipase, ausgewählt ist.

9. Verfahren nach einem der Ansprüche 1 bis 8, wobei das Gemisch mit dem Enzym in einer Lösung gemischt wird.

10. Verfahren nach einem der Ansprüche 1 bis 8, wobei das Gemisch durch eine Säule, welche das auf einem Träger immobilisierte Enzym enthält, durchgeleitet wird.

11. Verfahren nach einem der Ansprüche 1 bis 8, wobei die enzymatische Reaktion in der Gegenwart eines nicht-ionischen, grenzflächenaktiven Mittels stattfindet.

12. Verfahren nach Anspruch 11, wobei das nicht-ionische, grenzflächenaktive Mittel Triton X-100 ist.

13. Verfahren nach einem der Ansprüche 1 bis 12, welches weiter das Umkristallisieren des enantiomerisch angereicherten Gemisches umfaßt.

14. Verfahren nach einem der Ansprüche 1 bis 13, welches weiter das Behandeln des enantiomerisch angereicherten Gemisches mit einer chiralen Säure, ausgewählt aus der Gruppe, bestehend aus Apfelsäure, Mandelsäure, 10-Camphersulfonsäure, 3-Bromcampher-8-sulfonsäure, Dibenzoylweinsäure und Di-p-toluoylweinsäure, umfaßt.

15. Verfahren nach einem der Ansprüche 1 bis 14, welches weiter das Trennen des enantiomerisch angereicherten Gemisches unter Verwendung einer chiralen Säule umfaßt.

## Revendications

1. Procédé d'obtention de (-)-β-L-2-hydroxyméthyl-5-(5-fluorocytosin-1-yl)-1,3-oxathiolane, comprenant la résolution d'un mélange de (-)- et de (+)-cis-2-hydroxyméthyl-5-(5-fluorocytosin-1-yl)-1,3-oxathiolane par exposition du mélange à une cytidine-désoxycytidine-désaminase.

2. Procédé selon la revendication 1, comprenant en outre l'exposition du mélange énantiomériquement enrichi à une seconde enzyme pour résoudre en outre le (-)-β-L-2-hydroxyméthyl-5-(5-fluorocytosin-1-yl)-1,3-oxathiolane.

3. Procédé selon la revendication 1 ou la revendication 2, dans lequel le cis-2-hydroxyméthyl-5-(5-fluorocytosin-1-yl)-1,3-oxathiolane est acylé en position C5'-hydroxyle avant l'étape de résolution.

4. Procédé selon la revendication 3, dans lequel le cis-2-hydroxyméthyl-5-(5-fluorocytosin-1-yl)-1,3-oxathiolane est acylé avec un composé choisi dans le groupe constitué par les acides alkylcarboxyliques et les acides alkylcarboxyliques substitués.

5. Procédé selon la revendication 4, dans lequel l'acide alkylcarboxylique est choisi dans le groupe constitué par l'acide acétique, l'acide propionique, l'acide butyrique, l'acide pentanoïque, l'acide 2-chloropropionique, l'acide 2-chlorobutyrique et l'acide 2-chloropentanoïque.

6. Procédé selon l'une quelconque des revendications 2 à 5, dans lequel la seconde enzyme est choisie dans le groupe constitué par une estérase, une lipase, une subtilisine ou une α-chymotrypsine, qui catalyse préférentiellement une réaction dans l'un des énantiomères.

7. Procédé selon la revendication 6, dans lequel l'estérase est une estérase de foie de porc.

8. Procédé selon la revendication 6, dans lequel la lipase est choisie dans le groupe constitué par une lipase pancréatique porcine et la lipase Amano PS-800.

9. Procédé selon l'une quelconque des revendications 1 à 8, dans lequel le mélange est mélangé avec l'enzyme en solution.

10. Procédé selon l'une quelconque des revendications 1 à 8, dans lequel on fait passer le mélange à travers une colonne qui comprend l'enzyme immobilisée sur un support.

11. Procédé selon l'une quelconque des revendications 1 à 8, dans lequel la réaction enzymatique a lieu en présence d'un agent tensioactif non ionique.

12. Procédé selon la revendication 11, dans lequel l'agent tensioactif non ionique est le Triton X-100.

13. Procédé selon l'une quelconque des revendications 1 à 12, comprenant en outre la recristallisation du mélange énantiomériquement enrichi.

14. Procédé selon l'une quelconque des revendications 1 à 13, comprenant en outre le traitement du mélange énantiomériquement enrichi avec un acide chiral choisi dans le groupe constitué par l'acide malique, l'acide mandélique, l'acide 10-camphosulfonique, l'acide 3-bromocampho-8-sulfonique, l'acide dibenzoyltartrique et l'acide di-p-toluoyltartrique.

15. Procédé selon l'une quelconque des revendications 1 à 14, comprenant en outre la résolution du mélange énantiomériquement enrichi en utilisant une colonne chirale.
